# FASCICULE DE BREVET EUROPEEN

(11) **EP 3 747 492 B1**
(45) Date de publication et mention de la délivrance du brevet: **09.08.2023**
(21) Numéro de dépôt: 19305727.0
(22) Date de dépôt: 05.06.2019
(51) Int. Cl.: A61M 16/04

(54) **RÉGULATEUR DE PRESSION POUR BALLONNET DE SONDE D'INTUBATION, SYSTÈME DE RESPIRATION COMPORTANT UN TEL RÉGULATEUR ET RÉCIPIENT COMPORTANT UNE CHAMBRE À VOLUME VARIABLE POUR UN TEL RÉGULATEUR**
DRUCKREGULATOR FÜR CUFF AN ENDOTRACHEALTUBEN, ATMUNGSSYSTEM, DAS EINEN SOLCHEN REGULATOR UMFASST, UND BEHÄLTER, DER EINE KAMMER MIT VARIABLEM VOLUMEN FÜR EINEN SOLCHEN REGULATOR UMFASST
PRESSURE REGULATOR FOR BALLOON OF AN INTUBATION CATHETER, BREATHING SYSTEM COMPRISING SUCH A REGULATOR AND VESSEL COMPRISING A CHAMBER WITH VARIABLE VOLUME FOR SUCH A REGULATOR

(43) Date de publication de la demande: 09.12.2020
(73) Titulaire: Leved, 75011 Paris (FR)
(72) Inventeur: LEVÊQUE, Edouard, 75011 Paris (FR)
(74) Mandataire: Tranvouez, Edern Morgan

(56) Documents cités:
- EP-A1- 3 466 470
- WO-A1-99/40960
- WO-A1-2013/139986
- FR-A1- 2 940 621
- US-A- 4 501 273

## Description

La présente invention concerne un régulateur de pression pour sonde d'intubation coopérant avec un système de respiration destiné à la ventilation d'un patient, ledit système comportant un tuyau principal ayant une première extrémité destinée à être insérée dans la trachée artère du patient et une seconde extrémité destinée à être raccordée à un appareil respirateur, un ballonnet annulaire gonflable d'étanchéité, qui entoure le tuyau principal au niveau de la première extrémité et au moins un tuyau auxiliaire ayant une première extrémité raccordée au ballonnet d'étanchéité et une seconde extrémité munie d'une valve pouvant être raccordée à une source d'un gaz comprimé pour le gonflage du ballonnet d'étanchéité, ledit régulateur de pression étant inséré entre ladite source de gaz et le ballonnet, régulateur comprenant un récipient dont la base est en contact avec un plan de base, formé à partir d'une chambre à volume variable, ayant au moins un premier orifice pour être raccordé à ladite source de gaz et un second orifice pour être connecté audit tuyau auxiliaire de telle façon que, en service, la chambre à volume variable et le ballonnet d'étanchéité soient en communication, régulateur comprenant un poussoir agissant sur la chambre pour exercer une certaine force au moyen d'une masse de pression pouvant se déplacer pour régler ladite pression du gaz contenu dans ladite chambre à volume variable, et par suite la pression du gaz contenu dans le ballonnet d'étanchéité.

L'invention concerne aussi également un système de respiration comportant un tel régulateur.

Il est aussi décrit une chambre à volume variable convenant pour un tel régulateur.

Il arrive que des patients qui se trouvent dans un état comateux et qui souffrent d'une insuffisance respiratoire doivent être impérativement ventilés. Pour cela, on utilise habituellement une machine appelée « respirateur » ou « machine à ventiler », qui a pour fonction d'envoyer sous pression un mélange d'oxygène et de gaz carbonique, éventuellement mélangé avec un gaz anesthésiant, dans les poumons du patient. Ce mélange gazeux et la machine vont suppléer aux mouvements musculaires qui permettent au patient d'inspirer et d'expirer.

Le mélange gazeux peut être insufflé dans la trachée-artère du patient au moyen d'un tuyau (tuyau principal) qui passe soit par la bouche, soit par le nez du patient et qui est usuellement appelé « sonde d'intubation endo-trachéale » ou « sonde d'intubation endo-nasale » selon le cas. Si le patient doit être ventilé sur une longue durée (plus de quinze jours) ou si l'intubation est rendue impossible par des problèmes d'anatomie ou de traumatisme de la face, les chirurgiens ont habituellement recours à une « canule de trachéotomie ». Cette canule, plus ou moins rigide, de forme coudée, est constituée, comme les sondes d'intubations précitées, par un tuyau (tuyau principal) qui est introduit dans la trachée-artère du patient en pratiquant une ouverture dans la trachée (trachéotomie).

Lorsque le patient est ventilé par une sonde d'intubation ou une canule de trachéotomie, il est impératif de fermer hermétiquement l'accès par lequel est envoyé le mélange gazeux afin que celui puisse gonfler la cage thoracique du patient et rester dans ses poumons assez longtemps pour que l'échange oxygène/gaz carbonique s'effectue. Pour cela, le tuyau principal de la canule de trachéotomie ou de la sonde d'intubation est usuellement pourvu, dans la région de son extrémité qui est introduite dans la trachée-artère, d'un petit ballon annulaire gonflable, appelé « ballonnet » qui entoure l'extrémité précitée du tuyau principal. Une fois que la canule ou la sonde a été mise en place, ce ballonnet est gonflé à l'air à l'aide d'un petit tuyau (tuyau auxiliaire) qui est en partie incrusté dans la matière du tuyau principal et qui permet le passage de l'air entre le ballonnet et l'extérieur. L'extrémité extérieure du tuyau auxiliaire est habituellement pourvue d'une valve (clapet anti-retour), à laquelle peut être raccordée une source d'air comprimé, comme par exemple, une poire en caoutchouc actionnable à la main ou une seringue pour le gonflage du ballonnet. Cette valve empêche l'air de ressortir du ballonnet après que celui-ci a été gonflé et que la poire de gonflage en caoutchouc ou la seringue a été enlevée. Pour remplir sa fonction d'étanchéité, le ballonnet doit être hermétiquement plaqué contre la trachée-artère du patient afin de retenir le mélange insufflé dans les poumons tout le temps de l'insufflation. De plus, si le ballonnet n'est pas assez gonflé, des sécrétions peuvent descendre vers les poumons et entraîner ainsi des risques d'infections. Il est donc indispensable que le ballonnet soit correctement appliqué contre la paroi trachéale.

Toutefois, la pression qu'exerce le ballonnet de la sonde d'intubation ou de la canule de la trachéotomie sur la paroi trachéale n'est pas sans conséquence. En effet, toute pression continue sur une partie quelconque du corps humain gêne la circulation du sang dans la partie concernée. Si la pression exercée par le ballonnet sur la paroi trachéale est trop importante, cette pression empêchera la circulation sanguine dans la zone de la paroi trachéale qui est en contact avec le ballonnet. Cette absence de circulation sanguine, si elle se prolonge, peut entraîner une nécrose de la zone précitée de la trachée. Cette nécrose peut elle-même provoquer une sténose de la trachée, c'est-à-dire que son diamètre se réduit et que la trachée se resserre autour du ballonnet. Bien que l'air soit un élément compressible, en cas de sténose et pour chaque fluctuation du diamètre de la trachée (lors de chaque insufflation et exsufflation), il se produit une surpression dans le ballonnet qui gêne l'irrigation sanguine des tissus dans cette zone de la trachée. En effet, le volume d'air contenu dans le ballonnet oppose une résistance au resserrement de la trachée et cette résistance se traduit par une pression accrue du ballonnet contre la paroi trachéale, ce qui amplifie le phénomène de non-vascularisation, donc les risques de nécrose et de sténose.

D'autre part, lors d'une anesthésie générale, le gaz utilisé pour l'anesthésie, usuellement du protoxyde d'azote, possède une composition moléculaire telle que le gaz insufflé par le tuyau principal dans les poumons peut migrer dans le ballonnet d'étanchéité à travers la paroi de celui-ci. En rentrant dans le ballonnet, le gaz augmente le volume de ce dernier et cet accroissement de volume provoque une augmentation de la pression exercée par le ballonnet sur la trachée. Là encore, il y a donc un risque d'absence de vascularisation de la zone de la paroi trachéale qui est en contact avec le ballonnet, d'où un risque de nécrose et de sténose de la trachée.

Quand les ballonnets sont correctement gonflés, les sécrétions oro-pharyngées, non aspirées par le personnel médical, sont retenues au-dessus du ballonnet qui les empêche de descendre dans les poumons.

Cependant, pendant les périodes où le ballonnet est insuffisamment gonflé, les sécrétions au-dessus du ballonnet s'écoulent dans les poumons. Ces écoulements répétés provoquent des infections pulmonaires chez ces patients, infections pulmonaires, qui sont la première cause de décès en réanimation.

De manière à résoudre ces problèmes, on a proposé dans le document de brevet WO 99/40960, un régulateur de pression pour sonde d'intubation, canule de trachéotomie ou instrument similaire qui permet de maintenir une pression constante et réglable de l'air contenu dans le ballonnet d'étanchéité de l'instrument, et sensiblement constante mais réglable sur une paroi dudit récipient pour régler la pression de l'air contenu dans ladite chambre à volume variable et par suite, la pression de l'air contenu dans le ballonnet d'étanchéité.

Un autre document EP 3 466 470 décrit un régulateur du même type indiqué dans le préambule.

Bien que donnant entière satisfaction, il a été estimé que ce genre de système était encombrant et pouvait gêner les déplacements du personnel soignant.

Pour éviter cette gêne, un tel régulateur est remarquable en ce que le plan de base est en position verticale lors de son fonctionnement et en ce que ladite masse de pression est rattachée à une tige pivotant selon sa longueur dans un plan vertical autour d'un axe de fixation pour agir, par l'intermédiaire dudit poussoir, sur ladite chambre.

Un avantage important apporté par l'invention est que le régulateur peut être mis sur le bord du lit ou accroché au rebord du respirateur, il ne nécessite pas un emplacement horizontal tel qu'une table pouvant encombrer la chambre du malade.

La description suivante, accompagnée des dessins ci-annexés, le tout donné à titre d'exemple non limitatif fera bien comprendre comment l'invention peut être réalisée Sur les dessins :
La figure 1 représente un régulateur conforme à l'invention faisant partie d'un système respirateur,
la figure 2 représente une coupe du régulateur conforme à l'invention,
la figure 3 représente une vue agrandie de la masse de pression,
la figure 4 montre une coupe du régulateur après une rotation de la tige.

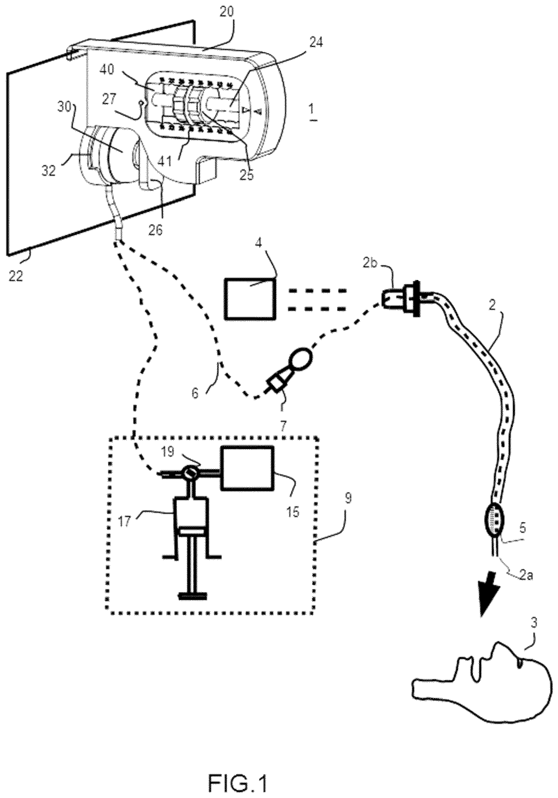

Comme on peut le voir à la figure 1 le régulateur de pression 1 fait partie d'un système respirateur pour insuffler de l'air par l'intermédiaire d'une canule de trachéotomie 2 à un patient 3. Cette canule se présente sous forme d'un tuyau, dit tuyau principal, de forme arquée constitué principalement en une matière élastomère flexible appropriée à une utilisation médicale ou chirurgicale et bien connue dans cette application de respiration assistée. Une première extrémité de ce tuyau 2a est destinée à être insérée dans la trachée artère du patient 3 et la seconde extrémité 2b est prévue pour être raccordée à un appareil respirateur d'un genre habituel représenté schématiquement par le bloc 4. Dans la région de son extrémité 2a, un ballonnet annulaire d'étanchéité 5 entoure ladite première extrémité 2a du tuyau principal 2, ledit ballonnet étant gonflable par l'intermédiaire d'un tuyau auxiliaire souple 6 ayant une première extrémité raccordée au ballonnet d'étanchéité 5 et une seconde extrémité rattachée à une valve anti-retour 7 qui est raccordée à une source d'air ou de gaz comprimé 9 comme par exemple une pompe actionnable à la main, telle qu'une poire en caoutchouc, pour le gonflage du ballonnet 5. Dans le cadre de cet exemple décrit, la source de gaz comprimé est formée d'un générateur d'air comprimé 15 et d'une seringue 17.

Le régulateur de pression 1 est intercalé entre la source d'air comprimé 9 et le tuyau auxiliaire 6. Le tuyau auxiliaire 6 est alimenté à partir de ces éléments 15 et 17 en fonction de la commande d'un robinet 19. La seringue 17 fournit l'air lors de la mise en route du régulateur. Il est à noter, pour des raisons de sécurité, que les différentes connexions sont effectuées en utilisant des tuyaux de couleurs différentes afin d'éviter des erreurs de connexions.

Selon un mode de réalisation de l'invention, le régulateur peut comporter un bâti 20 reposant sur un plan de base ou un support 22. Dans ce bâti 20 est placée une tige filetée 24 soutenant une masse de pression 25 qui peut se déplacer le long de cette tige. La tige 24 peut subir une rotation, selon sa longueur, dans un plan vertical. L'axe de cette rotation, qui est horizontal, porte la référence 27. La tige filetée 24 est solidaire d'un poussoir 26 qui rentre en contact avec une chambre à volume variable 30. Cette chambre, constituée par une matière plastique souple, est la partie essentielle d'un récipient 32 dont la base repose sur le fond du bâti 20 appuyé sur le support 22.

Selon l'invention, le support 22 est dans une position verticale de sorte que le poussoir 26 puisse exercer une pression variable en fonction du déplacement de la masse 25 sous l'effet de la pesanteur.

Ainsi, grâce à l'invention, il est possible de fixer le régulateur au lit d'un malade en l'accrochant au moyen de crochets ou même directement sur le respirateur.

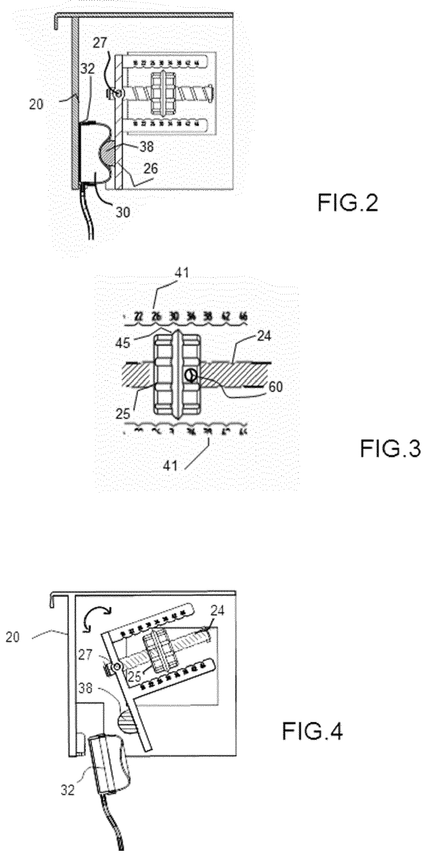

Selon un mode de réalisation, le bâti comporte une fenêtre 40 en regard de la masse 25 et de son déplacement possible. Sur les bords de cette fenêtre on a disposé des graduations 41, correspondant à la pression désirée. Un index 45 (voir figure 3) placé sur le pourtour de la masse 25 donne ainsi l'indication de la pression. Ceci est mieux montré dans les figures suivantes.

La figure 2 représente en coupe le bâti 20 conforme à l'invention. Il comprend le récipient 32 formé de la chambre à volume variable 30 subissant la pression du poussoir 26. Ce poussoir comporte une proéminence 38 qui se met en contact avec la chambre à volume variable 30.
La figure 3 montre une vue de la masse 25. Cette figure montre plus en détail les graduations 41 avec, en regard, l'index 45 placé sur le pourtour de la masse 25 de forme sensiblement cylindrique. Il est prévu un moyen de blocage du déplacement de ladite masse le long de la tige. Le moyen de blocage est constitué par une vis 60 insérée dans le corps de la masse de pression 25 pour rentrer en contact avec ladite tige 24 en vue dudit blocage.
La figure 4 montre qu'une rotation de la tige filetée autour de l'axe 27 permet de dégager le récipient 32 de sorte que l'on puisse changer commodément la chambre 30.

L'invention n'est bien entendu nullement limitée à l'exemple décrit donné à titre purement indicatif et que de nombreuses modifications peuvent être facilement apportées par l'homme de l'art sans pour autant sortir du cadre de l'invention.

## Revendications

1. Régulateur de pression (1) pour sonde d'intubation coopérant avec un système de respiration destiné à la ventilation d'un patient (3), ledit système comportant un tuyau principal (2) ayant une première extrémité (2a) destinée à être insérée dans la trachée artère du patient (3) et une seconde extrémité (2b) destinée à être raccordée à un _appareil respirateur (4), un ballonnet (5) annulaire gonflable d'étanchéité, qui entoure le tuyau principal (2) au niveau de la première extrémité (2a) et au moins un tuyau auxiliaire (6) ayant une première extrémité raccordée au ballonnet d'étanchéité (5) et une seconde extrémité munie d'une valve (7) pouvant être raccordée à une source d'un gaz comprimé (9) pour le gonflage du ballonnet d'étanchéité (5), ledit régulateur de pression étant inséré entre ladite source de gaz (9) et le ballonnet (5), régulateur comprenant un récipient (32) dont la base est en contact avec un plan de base (22), formé à partir d'une chambre à volume variable (30), ayant au moins un premier orifice pour être raccordé à ladite source de gaz (9) et un second orifice pour être connecté audit tuyau auxiliaire (6) de telle façon que, en service, la chambre à volume variable (30) et le ballonnet d'étanchéité (5) soient en communication, régulateur comprenant un poussoir (26) agissant sur la chambre (30) pour exercer une certaine force au moyen d'une masse de pression (25) pouvant se déplacer pour régler ladite pression du gaz contenu dans ladite chambre à volume variable, et par suite la pression du gaz contenu dans le ballonnet d'étanchéité (5), ladite masse de pression (25) étant rattachée à une tige (24) pivotant selon sa longueur dans un plan vertical autour d'un axe de fixation (27) pour agir, par l'intermédiaire dudit poussoir (26), sur ladite chambre (30) **caractérisé en ce que** ledit plan de base est disposé en position verticale, lors de son fonctionnement.

2. Régulateur selon la revendication 1 comportant un bâti (20) fixé dont une paroi constitue ledit plan de base et présentant une fenêtre (40) pour rendre accessible ladite masse (25) en vue de son déplacement le long de ladite tige (24) dont une extrémité est solidaire dudit poussoir (26).

3. Régulateur selon la revendication 2, dans lequel il est prévu au niveau de ladite fenêtre (40) des graduations (41) coopérant avec un index (45) faisant partie de ladite masse de pression (25) en vue de déterminer la pression à exercer sur ladite chambre (30).

4. Régulateur selon l'une des revendications 1 à 3 dans lequel il est prévu un moyen de blocage du déplacement de ladite masse le long de la tige.

5. Régulateur selon la revendication 4 dans lequel le moyen de blocage est constitué par une vis (60) insérée dans le corps de la masse de pression (25) pour rentrer en contact avec ladite tige (24) en vue dudit blocage.

6. Système de respiration comportant un régulateur selon l'une des revendications 1 à 5 et un appareil respirateur (4).

## Patentansprüche

1. Druckregler (1) für Intubationssonde, der mit einem Beatmungssystem zusammenwirkt, das zur Beatmung eines Patienten (3) bestimmt ist, wobei das System einen Hauptschlauch (2) mit einem ersten Ende (2a) aufweist, das zum Einsetzen in die Luftröhre des Patienten (3) bestimmt ist, und ein zweites Ende (2b), das zur Verbindung mit einem Beatmungsgerät (4) bestimmt ist, einen aufblasbaren ringförmigen Dichtballon (5), der den Hauptschlauch (2) im Bereich des ersten Endes (2a) umgibt und mindestens einen Hilfsschlauch (6) mit einem ersten Ende, das mit dem Dichtballon (5) verbunden ist und einem zweiten Ende, das mit einem Ventil (7) ausgestattet ist, das mit einer Druckgasquelle (9) zum Aufblasen des Dichtballons (5) verbindbar ist, wobei der Druckregler zwischen der Gasquelle (9) und dem Ballon (5) eingesetzt ist, wobei der Regler einen Behälter (32) umfasst, dessen Basis mit einer Basisebene (22) im Kontakt ist, der aus einer Kammer mit variablen Volumen (30) gebildet ist, mit mindestens einer ersten Öffnung, um mit der Gasquelle (9) verbunden zu sein, und einer zweiten Öffnung, um an den Hilfsschlauch (6) angeschlossen zu sein, so dass im Betrieb die Kammer mit variablem Volumen (30) und der Dichtballon (5) in Kommunikation sind, wobei der Regler einen Drücker (26) umfasst, der auf die Kammer (30) wirkt, um eine bestimmte Kraft mittels einer Druckmasse (25) auszuüben, die verlagerbar ist, um den Druck des Gases, das in der Kammer mit variablen Volumen enthalten ist und infolge den Druck des Gases, das im Dichtballon (5) enthalten ist, zu regeln, wobei die Druckmasse (25) einer Stange (24) zugeordnet ist, die gemäß ihrer Länge in einer vertikalen Ebene um eine Befestigungsachse (27) schwenkt, um über den Drücker (26) auf die Kammer (30) zu wirken, **dadurch gekennzeichnet, dass** die Basisebene im Betrieb in vertikaler Position angeordnet ist.

2. Regler nach Anspruch 1, der ein befestigtes Gestell (20) aufweist, von dem eine Wand die Basisebene bildet und ein Fenster (40) aufweist, um die Masse (25) zwecks ihrer Verlagerung entlang der Stange (24) zugänglich zu machen, von der ein Ende mit dem Drücker (26) fest verbunden ist.

3. Regler nach Anspruch 2, wobei im Bereich des Fensters (40) Skalen (41) vorgesehen sind, die mit einem Zeiger (45) zusammenwirken, der Teil der Druckmasse (25) ist, um den auf die Kammer (30) auszuübenden Druck zu bestimmen.

4. Regler nach einem der Ansprüche 1 bis 3, wobei ein Blockiermittel der Verlagerung der Masse entlang der Stange vorgesehen ist.

5. Regler nach Anspruch 4, wobei das Blockiermittel aus einer Schraube (60) besteht, die in den Körper der Druckmasse (25) eingesetzt ist, um mit der Stange (24) zwecks Blockade in Kontakt zu treten.

6. Beatmungssystem, das einen Regler nach einem der Ansprüche 1 bis 5 und ein Beatmungsgerät (4) aufweist.

## Claims

1. A pressure regulator (1) for an intubation probe cooperating with a breathing system intended for the ventilation of a patient (3), said system including a main pipe (2) having a first end (2a) intended to be inserted into the patient's trachea (3) and a second end (2b) intended to be connected to a breathing apparatus (4), an annular inflatable sealing balloon (5), which surrounds the main pipe (2) at the first end (2a) and at least one auxiliary pipe (6) having a first end connected to the sealing balloon (5) and a second end provided with a valve (7) which can be connected to a source of a compressed gas (9) to inflate the sealing balloon (5), said pressure regulator being inserted between said gas source (9) and the balloon (5), regulator comprising a container (32) whose base is in contact with a base plane (22), formed from a variable volume chamber (30), having at least one first orifice to be connected to said gas source (9) and a second orifice to be connected to said auxiliary pipe (6) such that, in use, the variable volume chamber (30) and the sealing balloon (5) are in communication, regulator comprising a tappet (26) acting on the chamber (30) to exert a certain force by means of a pressure mass (25) which can be displaced to adjust said pressure of the gas contained in said variable volume chamber, and consequently the pressure of the gas contained in the sealing balloon (5), said pressure mass (25) being attached to a rod (24) pivoting along its length in a vertical plane about a fixing shaft (27) to act, via said tappet (26), on said chamber (30) **characterised in that** said base plane is disposed in a vertical position, during its operation.

2. Regulator according to claim 1, including a fixed frame (20) of which one wall constitutes said base plane and having a window (40) to make said mass (25) accessible for the displacement thereof along said rod (24) of which one end is secured to said tappet (26).

3. Regulator according to claim 2, wherein graduations (41) cooperating with an index (45) forming part of said pressure mass (25) are provided at said window (40) in order to determine the pressure to be exerted on said chamber (30).

4. Regulator according to one of claims 1 to 3, wherein a means for blocking the displacement of said mass along the rod is provided.

5. Regulator according to claim 4, wherein the blocking means consists of a screw (60) inserted into the body of the pressure mass (25) to come into contact with said rod (24) for said blocking.

6. A breathing system including a regulator according to one of claims 1 to 5 and a breathing apparatus (4).
